# EUROPEAN PATENT APPLICATION

(11) **EP 2 278 334 A1**
(43) Date of publication of application: **26.01.2011**
(21) Application number: 09356049.8
(22) Date of filing: 22.07.2009
(51) Int. Cl.: G01N 33/543, B01L 3/00

(54) **A membrane based microarray device for high-throughput multianalyte detection**

(71) Applicant: Etablissement Francais du Sang, 93218 La Plaine Saint Denis Cedex (FR); UNIVERSITE CLAUDE BERNARD, 69100 Villeurbanne (FR); Centre National de la Recherche Scientifique, 75794 Paris Cedex 16 (FR); ECOLE NORMALE SUPERIEURE DE LYON, 69342 Lyon cedex 07 (FR)
(72) Inventor: Legoff, Gaëlle, 69006 Lyon (FR); Bres, Jean-Charles, 69007 lYON (FR); Marquette, Christophe, 69006 Lyon (FR); Rigal, Dominique, 69370 Lyon (FR); Blum, Loic, 69300 Caluire et Cuire (FR)
(74) Representative: Fiorucci, Hélène

(57) **Abstract**

A device - for performing an assay- comprising a substantially rigid support forming at least one well defining an inner cavity, having a bottom wall provided with a hole, a porous membrane sealed onto the support closing said hole and having an inner surface oriented towards the inside of the cavity and an opposite outer surface, at least one probe immobilized on the membrane, **characterized in that** the probe is immobilized outside the cavity and on the outer surface of the membrane.

## Description

The invention deals with a device - for performing an assay- comprising a substantially rigid support, a porous membrane sealed onto the support and at least one probe immobilized on the membrane. It also deals with a method of detection of an analyte using this device and the use of said method for genotyping blood cell antigens, quantitative antibody detection, early cancer diagnosis, protein/oligosaccharide interaction/detection or allergy diagnosis. The invention described hereinafter makes it possible to increase sensitivity, specificity, multidetection capacity and identification, while at the same time being rapid and easy to implement.

In a first aspect, the present invention deals with a device - for performing an assay- comprising a substantially rigid support forming at least one well defining an inner cavity, having a bottom wall provided with a hole, a porous membrane sealed onto the support closing said hole and having an inner surface oriented towards the inside of the cavity and an opposite outer surface, at least one detector probes immobilized on the membrane, **characterized in that** the probe is immobilized outside the cavity and on the outer surface of the membrane.

Many types of materials are suitable for the rigid support as long as they fulfill certain desirable characteristics. The main characteristic of the rigid support should be to conserve the characteristics of interaction of the capture probes to the analyte to be detected while at the same time generating a minimum background noise for the detection method (e.g. being chemically-resistant and biologically-inert).

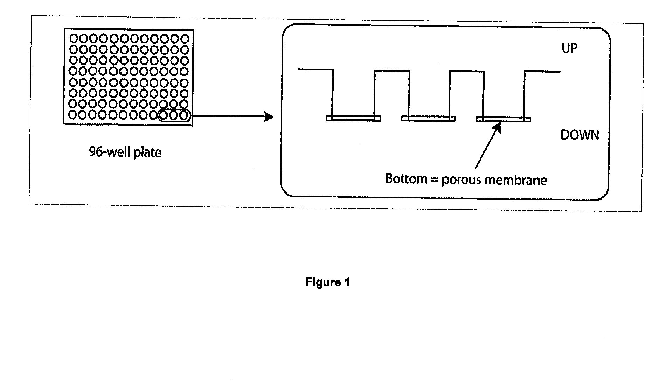

In one aspect of the invention, the device consists in a bottomless multi-well plate rigid support combined with a sealed porous membrane bottom and more particularly a bottomless 96-well plate (Figure 1).

According to another aspect of the present invention, the membrane is a porous polymeric membrane with possible surface activation capable of retaining and/or binding the probe. Examples of such membranes are:
- aldehyde activated *Ultrabind*® *membrane* from *Pall*, a modified polyethersulfone (PES) membrane that offers rapid covalent immobilization via an aldehyde chemistry.
- *Multiscreen HTS-HA* mixed cellulose ester membrane from *Millipore* (96-well plate design).

In a further aspect of the invention, the porous membrane is a flow-through permeable membrane.

Moreover due to the spotting configuration (spots outside the well) and the membrane porosity, this latter acts like a sample filter, which results in better signal/noise final ratios.

Assay strategy relies on interaction between these immobilized probes and analytes contained in the sample. Several types of probes can be arrayed on a single membrane enabling multiplexed analysis for one sample per well. Combining multiplexed analysis in each well and an 96 or more -well format leads to ultra-high throughput screening possibilities. On the contrary, one probe can be arrayed in several wells for enabling the analysis of one single system in many samples.

Each membrane (i.e. each well bottom) is functionalized with an array of probes molecules (i.e. a multitude of molecules are attached in predefined regions of the solid substrate to form a microarray). According to the present invention, a "probe" is immobilized or can be immobilized on the membrane by any suitable means.

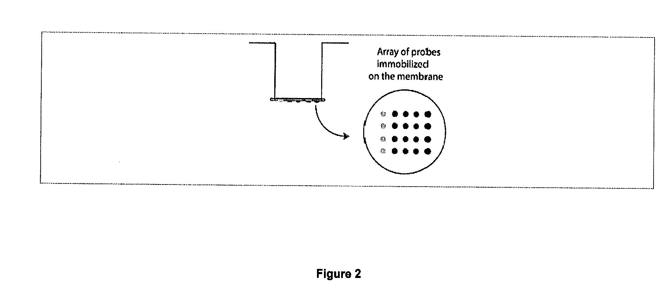

These probes are immobilized on the bottom side of the membrane (i.e. under the plate, not inside the well) (Figure 2).

The term "immobilized" on a porous membrane means according to the present invention that said probes are directly or indirectly fixed or immobilized to the substrate and remain associated with the substrate at least until all the manipulations are completed and the level of binding is assessed.

The expression "immobilized on a porous membrane" as used in the present specification refers to the attachment or adherence of one or more target-molecules to the surface of a porous substrate including attachment or adherence to the outer surface of said membrane.

Molecules or compounds may be immobilized either covalently (e.g., utilizing single reactive thiol groups of cysteine residues,) or non-covalently (adsorption) or specifically (e.g., via immobilized antibodies, the biotin/streptavidin system, and the like), by any method known in the art. Further examples of the various methods that are available to attach target-molecules to porous substrates include but are not limited to biotin-ligand non-covalently complexed with streptavidin, S-H-ligand covalently linked via an alkylating reagent such as an iodoacetamide or maleimide which reacts with SH and forms a thioether bond, amine-ligand covalently linked via an activated carboxylate group (e.g., EDAC coupled, etc.), phenylboronic acid (PBA)-ligand complexed with salicylhydroxamic acid (SHA), and acrylic linkages allowing polymerization with free acrylic acid monomers to form polyacrylamide or reaction with SH or silane surfaces. More specifically, immobilization of proteins may be accomplished through attachment agents selected from the group comprising cyanogen bromide, succinimides, aldehydes, tosyl chloride, avidin-biotin, photocrosslinkable agents including hetero bi-functional cross-linking agents such as N-[y-maleimidobutyryloxylsuccinimide ester (GMBS), epoxides, and maleimides. Antibodies may be attached to a porous substrate by chemically cross-linking a free amino group on the antibody to reactive side groups present within the support. For example, antibodies may be chemically crosslinked to a substrate that contains free amino, carboxyl, or sulfur groups using glutaraldehyde, carbo-di-imides, or hetero bifunctional agents such as GIVMS as cross-linkers.

Probes are designed to interact specifically with analytes of interest. Depending on the substrate's nature and the immobilization strategy (covalent, affinity...), probes can be functionalized for efficient immobilization.

Different probes may be arrayed in the mean time on a single membrane and matrix design may also include several replicates of a single probe.

No pre-treatment of the membrane surface is required; probes can be spotted directly on the membrane surface. Probes are diluted in a saline buffer (depending on the probe nature) and deposited as spots on the membrane surface using for example a non contact piezoelectric spotter. Drops size and thereby spots size (50-1000µm), spots density (1-10 per mm²), spot to spot distance (300 to 800 µm), probe concentration and matrix design can be modified depending on the application field and the probe nature.

After microarray spotting and eventually drying at room temperature (the duration of this step can be as short as 15 minutes but might be longer such as several days), a surface post-treatment can be carried out, including: washing steps (saline buffers...), chemical treatments (reduction...) and/or saturation steps (in order to minimize background signal).

The present invention provides arrays with probes that depend on the final use of the device. Probe molecules such as oligonucleotides, DNA, RNA, proteins toxins, antibiotics, antigens such as allergens or antibodies can be selected as probes depending on the application of interest thereby providing flexibility of selection of array content.

In the specific case of SNP detection, the length of the probe is of 15 to 30 nucleotides. The concentration of the spotting solution may vary from 10 to 50 µM, the spot to spot distance is comprised between 300 to 800 µm. Once spotted on the surface of the membrane, the membrane is dried at room temperature from 15 minutes to several days. The probes can be *in situ* synthesized on the solid support made such as the method described in U.S. Pat. No. 5,744,305 (Affymetrix, Fodor et al.) or EP 0061746 (University Patents Inc, Caruthers and Beaucage).After microarray spotting and drying at room temperature, surface post-treatment can proceed as following: membranes are washed by addition of an appropriate solution in each well, to eliminate probes in excess. The washing solutions can be applied either under continuous vacuum, by incubation and even the washing step can be omitted. A saturation step can also be carried out in order to avoid non specific bonding. Incubation can be carried out from 5 to 20 minutes at room temperature, 37, 45 or 50 °C.

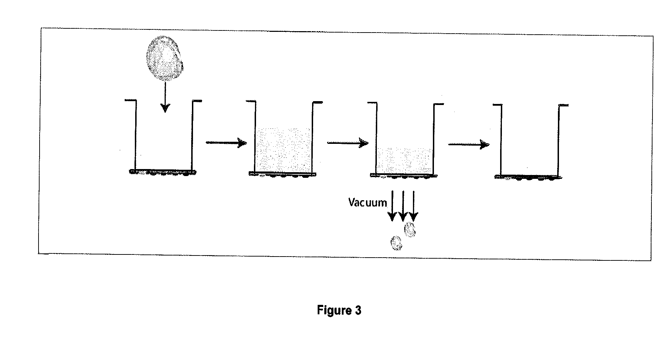

The membrane porosity enables solutions elimination through vacuum soaking. Protocols may involve sample incubation in well, as well as addition of a solution under continuous vacuum (Figure 3).

In a further aspect the invention deals with a method of preparation of a device consisting essentially of a substantially rigid support forming at least one well defining an inner cavity, having a bottom wall provided with a hole, a porous membrane sealed onto the support closing said hole and having an inner surface oriented towards the inside of the cavity and an opposite outer surface, at least one probe immobilized on the membrane comprising the step of immobilizing said probe outside the cavity and on the outer surface of the membrane.

In another aspect, the present invention deals with a method of determination of an analyte comprising the steps of:
(a) providing a device according to the present invention
(b) contacting said support with a sample liable to content at least an analyte under conditions allowing interaction of analyte with the detector probe to form analyte/detector probe complexes;
(c) optionally adding a detection labeled molecule detecting signals generated by said complexes.

"Determination" is understood to be the identification or the quantitative and/or qualitative detection or analysis of an analyte liable to be present in a sample. "Analyte" which may be employed in the present invention include, but are not limited to, antibodies including monoclonal antibodies polyclonal antibodies, purified antibodies, synthetic antibodies, antisera reactive with specific antigenic determinants (such as viruses, cells or other materials), proteins, peptides, polypeptides, enzyme binding sites, cell membrane receptors, lipids, proteolipids, drugs, polynucleotides, oligonucleotides, sugars, polysaccharides, cells, cellular membranes and organelles, nucleic acids including deoxyribonucleic acids (DNA), ribonucleic acids (RNA), peptide nucleic acids (PNA), locked nucleic acids (LNA), and aptamers (nucleic acids, proteins) or any combination thereof; cofactors, lectins, metabolites, enzyme substrates, metal ions and metal chelates.

The analytes may be naturally-occurring or man-made molecules. Also, they may be employed in their unaltered state or as aggregates with other species. Analytes may contain modifications, both naturally occurring or induced *in vitro* or *in vivo.* Induced modifications include adduct formation such as hapten attachment, multimerization, complex formation by interaction with other chemical moieties, digestion or cleavage (by, for example, protease), and metal ion attachment or removal. Analytes may be attached, covalently or non-covalently to, to a binding member, either directly or via a mediating linker.

"Interaction" is understood to be ligand-(membrane) receptor interactions; antigen-antibody interactions; protein-protein interactions; enzyme-substrate interactions; RNA-protein interactions; DNA-protein interactions; DNA-DNA interactions; cell-protein interactions.

A "sample" is any part obtained directly or indirectly from a starting product, matter or material, itself liable to contain at least one analyte. As a consequence of this definition, the sample to be determined in accordance with the present invention is liable to contain said analyte. For the purpose of the present invention, the starting product can be a biological material, a food or foodstuff, for example based on meat or fish, a cosmetic product, sera, blood, plasma, an environmental sample such as water, air and soil, etc.

Sample pre-treatment can be required (e.g. oligonucleotides sequences amplification, labelling or denaturation, purification, extraction, enzymatic digestion). Non-limiting examples of said pre-treatment include suspension/dilution of the sample in water or an appropriate buffer or removal of cellular debris, e.g. by centrifugation, or selection of particular fractions of the sample before analysis. Nucleic acid samples, for example, are typically isolated prior to assay and, in some embodiments, subjected to procedures, such as reverse transcription and/or amplification (e.g., polymerase chain reaction, mono to multiplex PCR) to increase the concentration of all sample nucleic acids (e.g., using random primers) or of specific types of nucleic acids (e.g., using polynucleotide-thymidylate to amplify messenger RNA or gene-specific primers to amplify specific gene sequences).

The conditions allowing interaction of the analyte to the probe depend on the nature of the analyte and of the probe.

The complexes formed between the analyte and the probe can either be detected directly or indirectly with a two-step method labeling, both of them implementing labeled molecules.

The term "label" as used in the present invention refers to a molecule propagating a signal to aid in detection and quantification. Said signal may be detected either visually (e.g., because it has color, or generates a colored product, or emits fluorescence) or by use of a detector that detects properties of the reporter molecule. In the present specification, labels allow for the detection of the interaction or association of two molecules. A non-limiting list of these tracers comprises chosen from enzymes (in particular a peroxidase, an alkaline phosphatase, or an enzyme capable of hydrolyzing a chromogenic, fluorigenic or luminescent substrate), chemical chromophore compounds, chromogenic, fluorigenic or luminescent compounds, nucleotide base analogs, and ligands such as biotin.

Useful labels in the present invention include for instance biotin for staining with labeled streptavidin conjugate, digoxigenin, anti-digoxigenin, luciferase, P-galactosidase, antigens, enzymes and enzyme conjugates, (e.g. horseradish peroxidase, alkaline phosphatase and others commonly used in e.g. ELISA). Particular suitable labels that may be employed in the invention may be chromogens including those that absorb light in a distinctive range of wavelengths so that a color may be observed or, alternatively, that emit light when irradiated with radiation of a particular wavelength or wavelength range, e.g., fluorescent molecules. Particular useful fluorescent labels include, by way of example and not limitation, fluorescein isothiocyanate (FITC), rhodamine, malachite green, Oregon green, Texas Red, Congo red, SybrGreen, phycoerythrin, allophycocyanin, 6-carboxyfluorescein (6-FAM), 2',7'-dimethoxy-4',5'-dichloro-6-carboxyfluorescein (JOE), 6-carboxy X-rhodamine (ROX), 6-carboxy-2',4',7',4,7-hexachlorofluorescein (HEX), 5-carboxyfluorescein (5-FAM), N,N,N',N'-tetramethyl-6-carboxyrhodamine (TAMRA), cyanine dyes (e.g. Cy5, Cy3), BODIPY dyes (e.g. BODIPY 630/650, Alexa542, etc), green fluorescent protein (GFP), blue fluorescent protein (BFP), yellow fluorescent protein (YFP), red fluorescent protein (RFP), and the like, (see, e.g., Molecular Probes, Eugene, Oreg., USA).

Any combination of labels, e.g. first and second labels, first, second, and third labels, etc. may be employed for analyte sets, provided the labels are distinguishable from one another. Examples of distinguishable labels are well-known in the art and include: two or more different wavelength fluorescent dyes, such as Cy3 and Cy5 or Alexa 542 and Bodipy 630/650; labels which generate signals under different treatment conditions, like temperature, pH treatment by additional chemical agents, etc.; and labels which generate signals at different time points after treatment.

Using one or more enzymes for signal generation allows for the use of an even greater variety of distinguishable labels based on different substrate specificity of enzymes (e.g. alkaline phosphatase/peroxidase).

Means for detecting such labels are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with an enzyme substrate and detecting the reaction product produced by the action of the enzyme on the substrate, and colorimetric labels are detected by simply visualizing the colored label.

Fluorescent labels are particularly suitable because they provide very strong signals with low background. Fluorescent labels are also optically detectable at high resolution and sensitivity through a quick scanning procedure. Fluorescent labels offer the additional advantage that irradiation of a fluorescent label with light can produce a plurality of emissions. Thus, a single label can provide for a plurality of measurable events.

Accordingly, in one embodiment of the present invention, a method is provided, wherein the detectable signal is an emitted light.

In a further embodiment of the present invention, a method is provided wherein the light emitted signal is a fluorescent signal or a color appearance. In yet a further embodiment of the present invention, a method is provided wherein a fluorescent signal is emitted by a labeled analyte, said analyte being associated with a target-molecule.

Desirably, fluorescent labels should absorb light above about 300 nm, usually above about 350 nm, and more usually above about 400 nm, usually emitting at wavelengths greater than about 10 nm higher than the wavelength of the light absorbed.

Detectable signal may equally be provided by chemiluminescent and bioluminescent labels. Chemiluminescent sources include compounds which becomes electronically excited by a chemical reaction and can then emit light which serves as the detectable signal or donates energy to a fluorescent acceptor. Alternatively, luciferins can be used in conjunction with luciferase or lucigenins to provide bioluminescence.

Analyte molecule(s) may be labeled before, during, or after the sample contacts the immobilized target-molecules by means of any method known in the art. "Label" according to the present invention can be an enzyme such as alkaline phopshatase, peroxidase or a nanoparticule.

So-called "direct labels" are detectable labels that are directly attached to or incorporated into the analyte molecule prior to interaction or binding with the cognate interaction or binding partner.

"Indirect labels" are attached to a component capable of binding to the analyte or to a member of a binding pair, the other member of which is attached to the analyte molecule. In indirect labeling, the labeled component may be linked to the analyte before, during or after the analyte containing sample contacts the immobilized target-molecules. Thus, for example, the analyte molecule(s) may be biotinylated and then bound to the cognate target-molecule binding partner. After binding, an avidin-conjugated fluorophore or avidin-conjugated nanoparticule may bind the biotin-bearing analyte molecule, providing a label that is easily detected. Labels may be attached to the analyte molecule(s) directly or through a linker moiety. In general, the site of label or linker-label attachment is not limited to any specific position. For example, in nucleic acid labeling, a label may be attached to a nucleoside, nucleotide, or analogue thereof at any position that does not interfere with detection or hybridization as desired.

Signal detection may be a quantitative or a qualitative observation or both. From the strength of the detected signals, the strength of the interaction events can be deduced and an interaction parameter may be calculated. Said interaction parameter may be in function of the amount of interaction between an immobilized target-molecule and an analyte.

In case of use of the optional step (c) in the method of determination of an analyte as cited above, (e.g. interaction between targets' biotin functionalities and streptavidin molecules) this one is carried out by:
1) Incubation of sample solution in wells (interaction probe/analyte)
2) Incubation of label molecules in (e.g. streptavidin conjugates) solution in wells

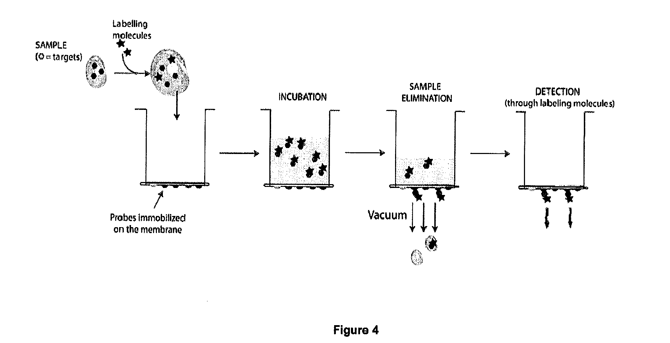

Step(c) may not be implemented (Figure 4), in this case, step (b) is carried out by incubation of a single premixed solution containing sample liable to contain analyte(s) and label molecules.

The following examples of the invention are exemplary and should not be taken as in way limiting.

### Brief description of the figures

**Figure 1****:** bottomless 96-well plate rigid support combined with a sealed porous membrane.
**Figure 2****:** membrane with molecules immobilized on its outer surface.
**Figure 3****:** elimination of the solution sample through vacuum soaking
**Figure 4****:** incubation of label molecules in solution in wells.
**Figure 5****:** colorimetric detection strategy.
**Figure 6****:** examples of resulting images (a) spotting map for one array (i.e. for one well) Spots are replicated 3 times per matrix. (b) to (e) pictures for four different samples (acquisition using an office scanner).
**Figure 7****:** Hybridization detection strategy
**Figure 8****:** image analysis and spot intensity quantification using GenePix Pro 6.0 software showing a correlation between target concentration and resulting signal intensity.
**Figure 9****:** antibodies detection strategy

### Example 1: Blood group genotyping (oligonucleotides microarrays)

### • Probes arraying

### Spotting

The twelve oligoprobes (see below) were diluted in phosphate buffer saline (phosphate 0.1M, NaCl 0.5M, pH=7.4) to reach a final concentration of 10µmol.L⁻¹, a concentration of 50µmol.L⁻¹ can also be used. The solutions were deposited as drops on the surface of an UltraBind® porous membrane (Pall) using a piezoelectric spotter (BioChip Arrayer BCA1, PerkinElmer). Probe length may vary between 15 and 30 nucleotides. No surface pre-treatment was required. The substrate was left at room temperature for drying. The drying time can be as short as 15 min but may last few days. Resulting spots diameters reached several hundreds of micrometers and spots density ranged from 1 to 10 per mm².

### Post-treatment

After microarray spotting and drying at room temperature, surface post-treatment proceeded as following: membranes were washed by addition of 1 mL PBS (added under continuous vacuum at room temperature) in each well. Arrays were then incubated at 50°C for 5 minutes with 200µL PBSTA (phosphate 0,1M, NaCl 0.5M, pH=7.4, Tween20 0.1\% v/v, BSA 1\% w/v) for saturation.

The reduction step using Sodium borohydrure is optional.

### Probe sequences for blood group SNPs screening: (Polymorphism is indicated with a bold character)

| **Blood group system** | **Allele** | **Sequence (5' to 3')** |
|---|---|---|
| **Kell** | **KEL1** | GTC TCA GCA TTC GGT TA- Ce NH₂ (SEQ ID No. 1) |
| | **KEL2** | TCT CAG CGT TCG GTT A- C₆ NH₂ (SEQ ID No. 2) |
| | **KEL3** | GGA ACA GCC ATG AAG TGA- C₆ NH₂ (SEQ ID No. 3) |
| | **KEL4** | GAA CAG CCG TGA AGT GA- C₆ NH₂ (SEQ ID No. 4) |
| **Kidd** | **JK1** | AGT AGA TGT CCT CAA ATG G- C₆ NH₂ (SEQ ID No. 5) |
| | **JK2** | AGT AGA TGT TCT CAA ATG G- C₆ NH₂ (SEQ ID No. 6) |
| **Duffy** | **FY1** | AGG TTG GCA CCA TAG TCT C- C₆ NH₂ (SEQ ID No. 7) |
| | **FY2** | GGT TGG CAT CAT AGT CT- C₆ NH₂ (SEQ ID No. 8) |
| **MNS** | **MNS3** | ATA GGA GAA ATG GGA CAA CT T- C₆ NH₂ (SEQ ID No. 9) |
| | **MNS4** | TAT AGG AGA AAC GGG ACA ACT TG- C₆ NH₂ (SEQ ID No. 10) |

### Biological samples preparation

Whole blood donors samples were obtained from the Etablissement Français du Sang Rhone-Alpes (Lyon) and were extensively phenotypes in the EFS Rhone-Alpes blood donations laboratory.

After genomic DNA extraction from blood sample (MagNA pure, Roche Diagnostics), twelve sequences hosting the six target polymorphisms were amplified and labeled with biotin using a single multiplexed PCR.

DNA samples were amplified under the following conditions on a Tprofessional thermal cycler (Biometra, Germany): 93°C for 5 min for enzyme activation followed by 35 cycles of 95°C for 20s, 57.5°C for 30s, 72°C for 30s. PCR reaction system contained: 10X PCR Buffer (150mM, Tris-HCl 500mM, KCI 0,1\%, Tween 20), MgCl₂ (25mM), dNTPs (2,5mM), Taq DNA Polymerase (5 units per µL) and specific primers in previously optimized concentrations. Forward primer was labelled with a biotin functionality at its 5' end.

PCR products were controlled using/performing gel electrophoresis (2\% agarose gel with 190V for 20min).

| **System** | **Allele** | **Sequence (5' to 3')** |
|---|---|---|
| Kell | KEL1/KEL2 | Biotin-GGT AAA TGG ACT TCC TTA AAC TTT AAC CGA (SEQ ID No. 11) |
| | | TGT GTC TTC GCC AGT GCA TC (SEQ ID No. 12) |
| | KEL3/KEL4 | Biotin-ACT CTT CCT TGT CAA TCT CCA TCA CTT (SEQ ID No. 13) |
| | | CAT GCC CAC AGT CTT CTG GC (SEQ ID No. 14) |
| Kidd | JK1/JK2 | Biotin-CTC AGT CTT TCA GCC CCA TTT GA (SEQ ID No. 15) |
| | | AGC GCC ATG AAC ATT CCT CC (SEQ ID No. 16) |
| Duffy | FY1/FY2 | Biotin-ATG ATT CCT TCC CAG ATG GAG ACT ATG (SEQ ID No. 17) |
| | | TGC CCA CAG CCA GCT GT (SEQ ID No. 18) |
| MNS | MNS3/MNS4 | Biotin-AGT GAA ACG ATG GAC AAG TTG TCC (SEQ ID No. 19) |
| | | GAT TCC AAA ATG ATT TTT TTC TTT GCA CAT GT (SEQ ID No. 20) |

### Assay

Without previous purification, PCR products underwent a 5 times dilution in PBSTA and were incubated at 95°C for 5min for deshybridization. Streptavidin-Alkalin Phosphatase conjugates were added to the previous mix in order to reach a final concentration of 1µg.mL⁻¹.

200µl of the resulting solution were added per well before incubation at 50°C for 30min.

Membranes were washed under continuous vacuum at room temperature with 1 mL of veronal saline buffer (veronal 0.1 M, NaCl 0.1 M, pH 8.5).

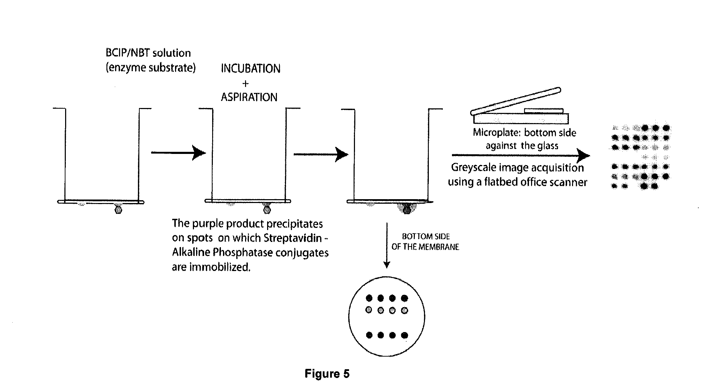

### Revelation (Figure 5)

100µL of a BCIP/NBT (4-bromo-5-chloroindolyl phosphate/ nitro-blue tetrazolium) solution were added in wells and plate was incubated at room temperature for signal revelation (around 30min). Membranes were washed with 1 mL PBS.

Once the membranes were dried, image was acquired using a flatbed office scanner (HP).

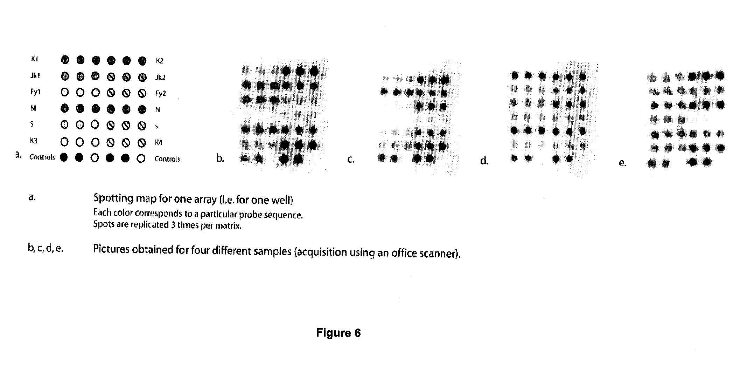

Examples of resulting images are showed (Figure 6).

### Image analysis

Image was analyzed and spots intensities were quantified using GenePix Pro 6.0 software (Molecular Devices). Signal intensities corresponding to two polymorphic alleles were compared by evaluating the following parameter: (I2-I1)/(I1+I2) where Iₙ refers to allele n intensity.

### Example 2. Quantitative oligonucleotide microarrays

Using a couple of synthetic oligonucleotides of complementary sequences as probe and target entities, it was showed that the present invention can be used for quantitative analysis of DNA sequences.

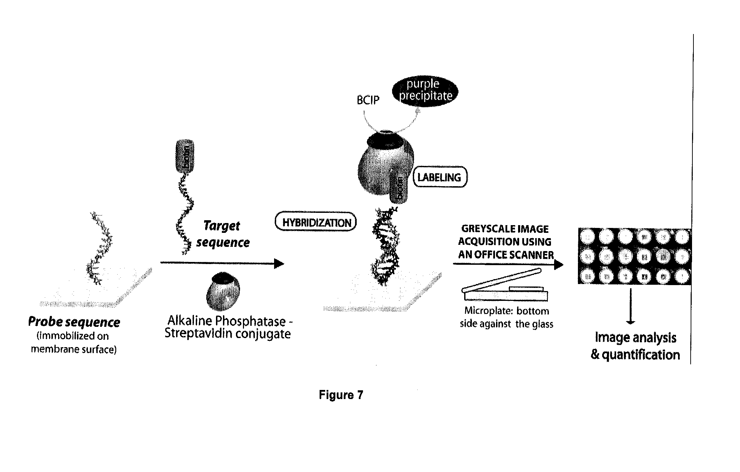

Probes were spotted on the membrane surface and hybridized with an oligonucleotide of complementary sequence. A biotin-streptavidin alkaline phosphatase indicator system was used for colorimetric detection (Figure 7)

### Probes arraying

The synthetic probe (TTG-AGG-TGC-ATG-TTT-GTG-CC, SEQ ID No. 21), 5' amino modification) was diluted in phosphate buffer saline (phosphate 0.1 M, NaCl 0.5M, pH=7.4) to reach the final concentration of 10µmol.L⁻¹. This solution was spotted on the surface of a Multiscreen HTS-HA membrane (Millipore) using a piezoelectric spotter (BioChip Arrayer BCA1, PerkinElmer). No surface pre-treatment was required. The substrate was left at room temperature for drying. Resulting spots diameters ranged around 200µm and spots density went from 1 to 10 per mm².

After microarray spotting and drying at room temperature, surface post-treatment proceeded as following: membranes were washed by addition of 1 mL PBS (added under continuous vacuum at room temperature in wells). They were then incubated at 50°C for 5 minutes with 200µL PBSTA (phosphate 0.1 M, NaCl 0.5M, pH=7.4, Tween20 0.1\% v/v, BSA 1\% w/v) for saturation.

### Assay

Solutions of target oligonucleotide at different concentrations and streptavidin-alkaline phosphatase conjugates (1µg.mL⁻¹) were prepared in PBSTA. 200µl of the resulting solution were dispensed in each well before incubation at 37°C for 30min.

Membranes were washed under continuous vacuum at room temperature with 1 mL of veronal saline buffer (veronal 0.1 M, NaCl 0.1 M, pH 8.5).

### Revelation

100µL of a BCIP/NBT (5- bromo-4-chloroindolyl phosphate/ nitro-blue tetrazolium) solution were added in wells and plate was incubated at room temperature for signal revelation (around 30min). Membranes were washed with 1 mL PBS. Once membranes were dried, image was acquired using a flatbed office scanner (HP).

### Image analysis

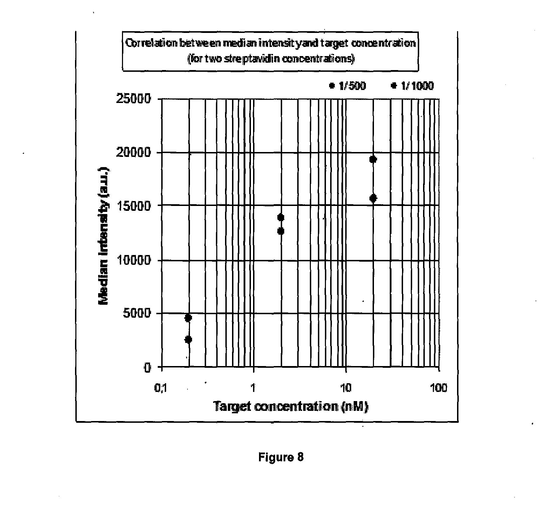

Image was analyzed and spots intensities were quantified using GenePix Pro 6.0 software (Molecular Devices) showing a correlation between target concentration and resulting signal intensity. (Figure 8)

### Example 3: Proteins arrays for quantitative antibodies detection for point of care diagnosis applications

When membranes are functionalized with protein microarrays, the present invention enables quantitative serologic tests, which could be performed for point-of-care diagnosis applications.

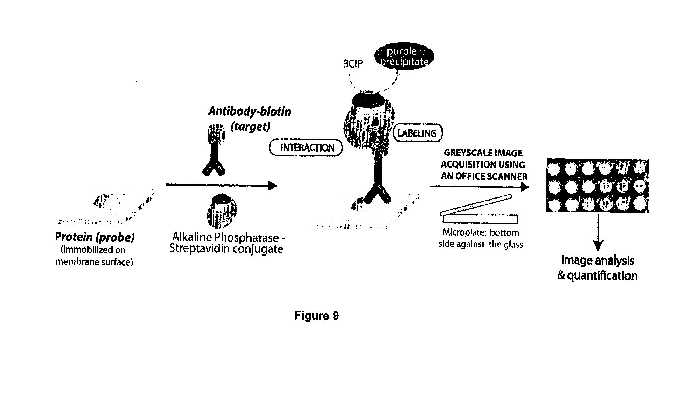

To evaluate the reliability of such quantitative assays, the following system was used: CRP molecules were immobilized on the membrane in order to act like probes for detection of the corresponding biotin-antibody targets. A streptavidin-alkaline phosphatase indicator system was used for colorimetric detection. A correlation between signal intensity and target antibody concentration was demonstrated. (Figure 9).

### Probes arraying

A solution of 150µg.mL⁻¹ of CRP in phosphate buffer saline (phosphate 0.1 M, NaCl 0.5M, pH=7.4) was spotted on the surface of a Multiscreen HTS-HA membrane (Millipore) using a piezoelectric spotter (BioChip Arrayer BCA1, PerkinElmer). No surface pre-treatment was required. The substrate was left for 30 minutes at room temperature for drying. Resulting spots diameters ranged around 200µm and spots density went from 1 to 10 per mm².

After microarray spotting and drying at room temperature, surface post-treatment proceeded as following: membranes were washed by addition of 1 mL veronal buffer saline containing calcium VBS-Ca (veronal 0,1 M, NaCl 0,5M, Ca²⁺ 2mM, pH=8.5) added under continuous vacuum at room temperature in wells. Arrays were then incubated at 50°C for 5 minutes with 200µL VBSTA (veronal 0,1M, NaCl 0,5M, Ca²⁺ 2mM, pH=8.5, Tween20 0,1\% v/v, BSA 1\% w/v) for saturation.

### Assay

Mixed solutions of target biotin-antibody (several concentrations) and streptavidin-alkaline phosphatase conjugates (1µg.mL⁻¹) were prepared in VBSTA-Ca. 200µl of the resulting solution were dispensed in each well before incubation at 37°C for 30min.

Membranes were washed under continuous vacuum at room temperature with 1 mL VBS-Ca.

### Revelation

100µL of a BCIP/NBT (5-bromo-4-chloroindolyl phosphate / nitro-blue tetrazolium) solution were added in wells and the plate was incubated at room temperature for signal revelation (around 30min). Membranes were washed with 1mL PBS.

Once membranes were dried, image was acquired using a flatbed office scanner (HP).

### Image analysis

Image was analyzed and spots intensities were quantified using GenePix Pro 6.0 software (Molecular Devices) showing a correlation between resulting signal intensity and target antibodies concentration.

This one-step protocol can be adapted to a sandwich assay (implying then a secondary antibody).

### Example 5. Protein arrays for serology for early cancer diagnosis

Autoimmune antibodies presence in serum can be an evidence of cancer apparition.

An early cancer diagnosis tool based on detection of such autoimmune antibodies was developed using the present invention.

This serologic test consists in a capture assay of these target autoimmune antibodies using protein microarrays and a colorimetric detection (alkaline phosphatase - anti-human antibody conjugates indicator system). The autoimmune antibodies in serum interact with proteins immobilized on the membrane and anti-human antibody conjugated with alkaline phosphatase is used to detect also captured target antibodies.

This strategy can also be applied for allergy diagnosis. In that case, IgE (targets) interact with specific allergens (probes) immobilized on the membrane.

## Claims

1. A device - for performing an assay- comprising :
- A substantially rigid support forming at least one well defining an inner cavity, having a bottom wall provided with a hole,
- A porous membrane sealed onto the support closing said hole and having an inner surface oriented towards the inside of the cavity and an opposite outer surface,
- At least one probe immobilized on the membrane,
**characterized in that** the probe is immobilized outside the cavity and on the outer surface of the membrane.

2. A device according to claim 1 **characterized in that** the porous membrane is a flow-through permeable membrane.

3. A device according to claim 2 **characterized in that** the porous membrane is a polymeric membrane such as polyethersulfone or mixed cellulose membranes.

4. A device according to any of the preceding claims wherein the detector probes are spatially arranged to form microarrays

5. A device according to claim 1 **characterized in that** the detector probes are oligonucleotides, such as DNA or RNA, peptides, oligosaccharides, proteins or antibodies.

6. A device according to claim 3 **characterized in that** the probes are functionalized.

7. A method of detection of an analyte comprising the steps of :
(a) providing a device according to claims 1 to 6
(b) contacting said device with a sample liable to content at least an analyte under conditions allowing interaction of analyte to the probe to form analyte/detector probe complexes;
(c) optionally adding a detection labeled molecule
(d) detecting signals generated by said complexes from the bottom or from the top of the cavity.

8. A method of detection of an analyte according to claim 7 in which the sample liable to content at least the analyte is the unpurified solution resulting of a multiplex PCR.

9. A method of detection according to claim 7 wherein the detection of the signal is made by colorimetry, fluorescence or chemiluminescence.

10. A method of detection according to claim 7 to 9 in which the analyte originates from a biological sample such as serum, blood or plasma, an environmental sample such as water, gas, air and soil or a sample from food industry such as food or food stuff.

11. Use of a method according to claim 7 or 8 for genotyping blood cell antigens, quantitative antibody detection, early cancer diagnosis protein/oligosaccharide interaction detection or allergy diagnosis.

12. A method of preparation of a device consisting essentially of a substantially rigid support forming at least one well defining an inner cavity, having a bottom wall provided with a hole, a porous membrane sealed onto the support closing said hole and having an inner surface oriented towards the inside of the cavity and an opposite outer surface, at least one probe immobilized on the membrane comprising the step of immobilizing said probe outside the cavity and on the outer surface of the membrane.


SEQUENCE LISTING

<110>  Etablissement Français du Sang
       Université Claude Bernard Lyon 1
       Ecole Normale Supérieure de Lyon
       Centre National de la Recherche Scientifique

<120>  A membrane based microarray device for high-throughput
       multianalyte detection

<130>  BR063632

<160>  21

<170>  PatentIn version 3.5

<210>  1
<211>  17
<212>  DNA
<213>  Artificial Sequence

<220>
<223>  Probe sequence for blood group SNPs screening

<400>  1
gtctcagcat tcggtta                                                    17


<210>  2
<211>  16
<212>  DNA
<213>  Artificial Sequence

<220>
<223>  Probe sequence for blood group SNPs screening

<400>  2
tctcagcgtt cggtta                                                     16


<210>  3
<211>  18
<212>  DNA
<213>  Artificial Sequence

<220>
<223>  Probe sequence for blood group SNPs screening

<400>  3
ggaacagcca tgaagtga                                                   18


<210>  4
<211>  17
<212>  DNA
<213>  Artificial Sequence

<220>
<223>  Probe sequence for blood group SNPs screening

<400>  4
gaacagccgt gaagtga                                                    17


<210>  5
<211>  19
<212>  DNA
<213>  Artificial Sequence

<220>
<223>  Probe sequence for blood group SNPs screening

<400>  5
agtagatgtc ctcaaatgg                                                    19


<210>  6
<211>  19
<212>  DNA
<213>  Artificial Sequence

<220>
<223>  Probe sequence for blood group SNPs screening

<400>  6
agtagatgtt ctcaaatgg                                                    19


<210>  7
<211>  19
<212>  DNA
<213>  Artificial Sequence

<220>
<223>  Probe sequence for blood group SNPs screening

<400>  7
aggttggcac catagtctc                                                    19


<210>  8
<211>  17
<212>  DNA
<213>  Artificial Sequence

<220>
<223>  Probe sequence for blood group SNPs screening

<400>  8
ggttggcatc atagtct                                                      17


<210>  9
<211>  21
<212>  DNA
<213>  Artificial Sequence

<220>
<223>  Probe sequence for blood group SNPs screening

<400>  9
ataggagaaa tgggacaact t                                                 21


<210>  10
<211>  23
<212>  DNA
<213>  Artificial Sequence

<220>
<223>  Probe sequence for blood group SNPs screening

<400>  10
tataggagaa acgggacaac ttg                                               23

<210>  11
<211>  30
<212>  DNA
<213>  Artificial Sequence

<220>
<223>  Probe for PCR

<400>  11
ggtaaatgga cttccttaaa ctttaaccga                                        30


<210>  12
<211>  20
<212>  DNA
<213>  Artificial Sequence

<220>
<223>  Probe for PCR

<400>  12
tgtgtcttcg ccagtgcatc                                                   20


<210>  13
<211>  27
<212>  DNA
<213>  Artificial Sequence

<220>
<223>  Probe for PCR

<400>  13
actcttcctt gtcaatctcc atcactt                                           27


<210>  14
<211>  20
<212>  DNA
<213>  Artificial Sequence

<220>
<223>  Probe for PCR

<400>  14
catgcccaca gtcttctggc                                                   20


<210>  15
<211>  23
<212>  DNA
<213>  Artificial Sequence

<220>
<223>  Probe for PCR

<400>  15
ctcagtcttt cagccccatt tga                                               23


<210>  16
<211>  20
<212>  DNA
<213>  Artificial Sequence

<220>
<223>  Probe for PCR

<400>  16
agcgccatga acattcctcc                                                    20


<210>  17
<211>  27
<212>  DNA
<213>  Artificial Sequence

<220>
<223>  Probe for PCR

<400>  17
atgattcctt cccagatgga gactatg                                            27


<210>  18
<211>  17
<212>  DNA
<213>  Artificial Sequence

<220>
<223>  Probe for PCR

<400>  18
tgcccacagc cagctgt                                                       17


<210>  19
<211>  24
<212>  DNA
<213>  Artificial Sequence

<220>
<223>  Probe for PCR

<400>  19
agtgaaacga tggacaagtt gtcc                                               24


<210>  20
<211>  32
<212>  DNA
<213>  Artificial Sequence

<220>
<223>  Probe for PCR

<400>  20
gattccaaaa tgattttttt ctttgcacat gt                                      32


<210>  21
<211>  20
<212>  DNA
<213>  Artificial Sequence

<220>
<223>  synthetic probe

<400>  21
ttgaggtgca tgtttgtgcc                                                    20